Europäisches Patentamt

(19) **European Patent Office**

**Office européen des brevets**

(11) Publication number: **0 150 281**
**A1**

(12) # EUROPEAN PATENT APPLICATION

(21) Application number: **84113474.5**

(22) Date of filing: **08.11.84**

(51) Int. Cl.⁴: **A 61 M 25/00**

---

(30) Priority: **15.11.83 US 551860**

(43) Date of publication of application: **07.08.85**
**Bulletin 85/32**

(84) Designated Contracting States: **DE FR GB IT**

(71) Applicant: **MEDI-TECH, INCORPORATED, 480 Pleasant Street, Watertown Massachusetts 02172 (US)**

(72) Inventor: **Gould, Arnold S., Three Clark Road, Bedford Massachusetts 01730 (US)**
Inventor: **Ciannella, Michael A., 14 Spring Hill Avenue, Marlboro Massachusetts 01752 (US)**

(74) Representative: **Heidrich, Udo, Dr. jur., Dipl.-Phys., Franziskanerstrasse 30, D-8000 München 80 (DE)**

---

(54) **Medical introducer-catheter system.**

㉝ A medical catheter (14) constructed for use with a preselected trocar (12) having a trocar head (20) larger than an adjoining neck (26) and supporting shaft (18). The catheter has a distal end portion (16) defining an aperture (36) smaller than the diameter of the shaft, the distal end portion being configured and adapted to elastically expand in diameter to permit passage of the trocar head therethrough and to elastically contract to engage about the neck of the trocar. The outer diameter of the portion of the catheter engaged about the neck of the trocar has about the same diameter as the proximal end of the trocar head so that there is no movement-impeding protrusion at the transition between the outer surfaces of the trocar head and the distal portion of the catheter.

RECHTSANWALT & PATENTANWALT
DIPL.-PHYS. DR. JUR. U. HEIDRICH
Franziskanerstr. 30
D - 8000 MÜNCHEN 80

November 8, 1984
MEDI-TECH, INC.
Introducer

– /1 –

## MEDICAL INTRODUCER-CATHETER SYSTEM
### Background of the Invention

The invention relates to catheters and the like and to instruments for introducing such devices into the body.

In some procedures, to introduce conduit-defining catheters into the body of a patient by percutaneous entry, a trocar or needle is passed coaxially through the catheter, with its point extending distally for penetration of tissue. The trocar or needle is sized to closely approximate the inner diameter of the catheter, but it has seemed unavoidable to have the lead edge of the catheter protrude laterally to the side. Such protrusion can hinder smooth penetration and has been known to cause trauma to the surrounding tissue, bleeding and discomfort.

It has been suggested to feather (taper) the wall of the catheter at the tip to reduce the severity of the protrusion created, however, this has weakened the wall, allowing it to roll and kink during insertion.

Insertion is particularly a problem into organs such as the stomach, which tend to be flacid and move away from external puncture forces applied with the trocar.

### Summary of the Invention

A medical catheter or the like comprised of elastomeric material is constructed for use with a preselected trocar having a trocar head larger than an adjoining neck and supporting shaft, the catheter having a distal end portion defining an aperture smaller than the diameter of the shaft, the distal end portion configured and adapted to elastically expand in diameter to permit passage of the trocar head therethrough and to elastically contract to engage about the neck of the trocar, the outer diameter of the portion of the catheter engaged about the neck of the trocar being about the same as the diameter of the proximal end of the trocar head so that there is no movement-impeding protrusion at the transition between the outer surfaces of the trocar head and the distal portion of the catheter.

In preferred embodiments, the end portion of the catheter is specially shaped to substantially fill the space surrounding the neck region of the trocar in the manner to provide a smoothly formed transition from the exterior surface of the head to the exterior surface of the catheter; the wall of its distal end portion has at least one longitudinal slit to facilitate the expansion of the distal portion and withdrawal of the trocar head therethrough; and the distal end portion of the catheter is comprised of at least two cylindrical layers in overlying relationship, one the layer being elastic, preferably the inner of the cylindrical layers is of relatively stiff substance, and is slit longitudinally at least at one point about its circumference to enable expansion of the catheter neck to permit passage of the head of the trocar therethrough, and the elastic layer is continuous and overlies the region of the slit.

In one special aspect, the medical catheter is provided in combination with a trocar or the like, the combination being adapted for introduction into the body of a person, the trocar comprised of an elongated, rigid shaft sized to extend coaxially through the catheter, the trocar having a generally pointed head at its distal end, and a reduced size portion immediately proximal of the head, whereby, during insertion of the catheter by means of the trocar, there is no movement-impeding protrusion at the transition between the trocar head and the distal portion of the catheter.

### Preferred Embodiment

We first briefly describe the drawings.

### Drawings

Fig. 1 is a side view partially in section of a preferred embodiment according to the invention, including a trocar with a necked region proximal of its pointed head and a matching catheter;

Fig. 1a is a perspective view of the distal end of the preferred catheter of Fig. 1;

Figs. 2, 3 and 4 are views respectively showing the relationship of the expansible-contractible catheter tip to the trocar during insertion of the trocar into the catheter, percutaneous insertion of the trocar-and-catheter unit into the patient's body, and withdrawal of the trocar from the catheter positioned within the patient's body; and

Fig. 5 is a side section view showing another preferred embodiment of the invention, with a specially molded sheath tip engaged about a needle.

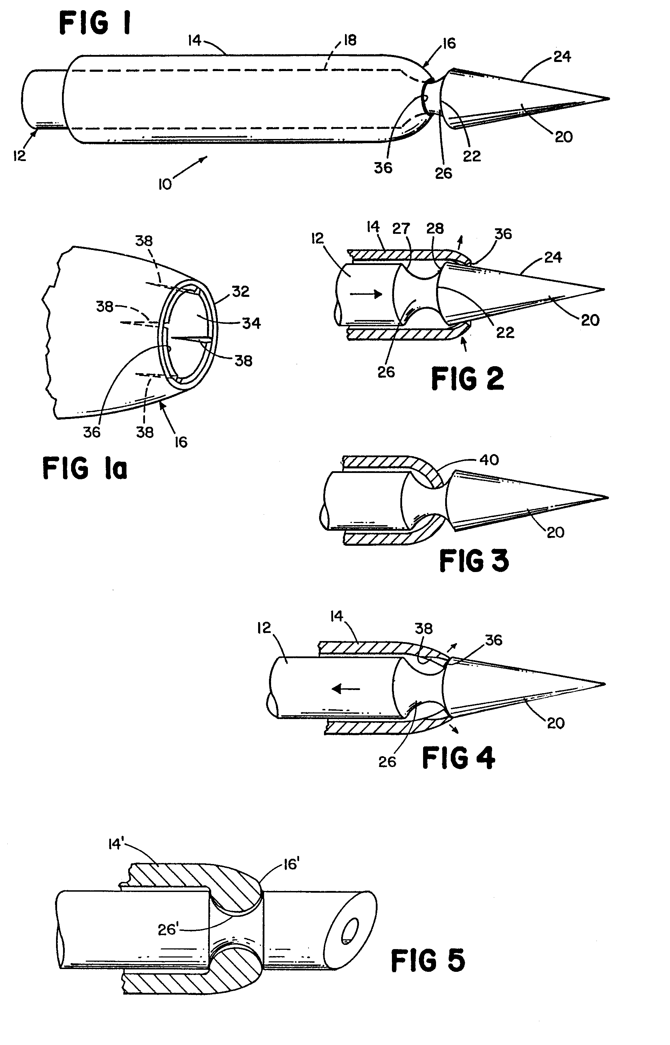

Referring to Fig. 1, medical device 10 comprises matching trocar 12 and catheter 14.

Trocar 12, typically stainless steel, comprises a rigid shaft 18, of approximately 0.109 inch diameter, and a puncturing head 20. Head 20 is generally conical in shape, pointed tip directed distally, and has the same diameter at the proximally-directed base 22 as shaft 18, i.e. 0.109 inch. (By shaft and head having the same diameter the trocar can be formed by single machining operations from cylindrical stock.) The head is about 3/16 inch long. Surface 24 of head 20 is smooth to facilitate passage through the patient's flesh and lies at an angle of about 10 degrees to the trocar axis to provide gentle spreading of the tissue about the puncturing tip to form a passage for the catheter being introduced. Defined between shaft 18 and head 20 is neck 26, an annular groove about 0.050 inch across and 0.025 inch deep at the center on a 0.025 inch radius. This groove extends circumferentially about the trocar. The surface 28 of the distal portion of the groove 26, flaring outwardly to the trocar axis, provides a camming surface to facilitate removal of the trocar from the catheter within the body, as discussed more fully below. At the proximal end, the surface 27 of the groove, having a distally-directed component, enables application of increased insertion force to the end of the catheter by the trocar.

Referring now to Fig. 1a, the catheter 14 shown is double walled. The outer tube 32 is 14 French, i.e. about 0.182 inch diameter, with a wall thickness of about 0.010 inch. To provide elasticity, the tubing is of low durometer, e.g. about 80A, polyurethane. The inner tube 34 is typically relatively stiff, having a wall thickness of about 0.015 inch and durometer of about 70D. Both tubes extend to the distal tip 16 of the catheter where they are joined and somewhat narrowed. The inner tube 34 is slit longitudinally at 38 from the distal end, e.g. at four points, 90 degrees to each other, to allow expansion of neck aperture 36 over the trocar head. The outer layer of tubing 32, overlying the slits 38 in the wall of the inner tubing 34, allowing the expansion, provides a contracting force to engage the catheter about the trocar neck 26 and presents a smooth surface to the exterior.

Referring now to Figs. 2 through 4, the relationship of the trocar 12 and catheter 14 of system 10 is shown. In Fig. 2, trocar 12 is shown being inserted coaxially through catheter 14, with the trocar moving distally, i.e. to the right as shown, relative to the catheter. As trocar head 20 reaches the narrowed tip 16 of catheter 14, the inner surface of the catheter defining the aperture 36 slides along surface 24 of the trocar head, expanding elastically to facilitate passage of the head therethrough. When the wide base 22 of the head passes through the catheter tip, the tip contracts about the neck 26 of the trocar (Fig. 3). Thus assembled, with the catheter tip engaged about the trocar neck, the trocar-and-catheter unit may be inserted percutaneously into the patient's body. As seen in Fig. 3, the tip of the catheter lies radially inward of the proximal base 22 of the trocar head 20, with no movement-impeding protrusion or exposed butt surface that might otherwise hinder insertion or cause trauma to the surrounding tissue. (As long as the diameter of the surface of the catheter tip is substantially the same as the diameter of the base of the trocar head, and there is no or only slight protrusion, benefit of the invention is realized.) Any increase in diameter caused by the thickness of the catheter wall is introduced gradually, at the proximal end of the neck region, by the smooth outer wall surface 40 of the catheter, without any trauma-causing protrusion. By this construction the end of the catheter resists rolling or kinking during insertion.

In Fig. 4, the catheter 14 has been properly positioned in the patient's body, and the trocar 12 is being withdrawn in the proximal direction relative to the catheter, i.e. to left as shown. The distal tip 16 of the catheter engaged about the trocar neck 26 slides along camming surface 28, which is disposed at an acute angle to the trocar axis to urge the catheter aperture 36 to expand to allow the trocar head 20 to pass rearwardly through the aperture, without dislodging the catheter 14.

The trocar is then withdrawn from the catheter and the catheter contracts to its original diameter to be employed in the desired medical procedure.

## Other Embodiments

Other embodiments are within the following claims. For example, as shown in Fig. 5, a catheter or sheath 14' having a tip 16' specially formed to tightly correspond to the groove 26' may be provided. (In the figure, a needle is shown.) Also, the trocar or needle may be employed with other catheters or the like having distal apertures of appropriate size and sufficient elasticity. In some cases the diameter of the body of the trocar may be smaller than the largest diameter of the head.

CLAIMS

1. A medical catheter 14 or the like comprised of elastomeric material,

characterized in that

said catheter is constructed for use with a preselected trocar 12 having a trocar head larger than an adjoining neck 26 and supporting shaft 18,

said catheter having a distal end portion 16 defining an aperture 36 smaller than the diameter of said shaft, said distal end portion configured and adapted to elastically expand in diameter to permit passage of said trocar head therethrough and to elastically contract to engage about the neck of said trocar,

the outer diameter of the portion of said catheter engaged about the neck of said trocar being about the same as the diameter of the proximal end of the trocar head so that there is no movement-impeding protrusion at the transition between the outer surfaces of said trocar head and the distal portion of said catheter.

2. The catheter of claim 1
characterized in that
the end portion of said catheter is specially shaped to substantially fill the space surrounding the neck region of said trocar in the manner to provide a smoothly formed transition from the exterior surface 24 of said head to the exterior surface of said catheter.

3. The catheter or the like of claim
characterized in that
the wall of its distal end portion has at least one longitudinal slit 38 to facilitate the expansion of said distal portion and withdrawal of said trocar head 20 therethrough.

4. The catheter or the like of claim 1 characterized in that the distal end portion 16 of said catheter is comprised of at least two cylindrical layers 32, 34 in overlying relationship, one said layer being elastic.

5. The catheter or the like of claim 4 characterized in that the inner 34 of said cylindrical layers is of relatively stiff substance, and is slit longitudinally 38 at least at one point about its circumference to enable expansion of said catheter neck 26 to permit passage of the head 20 of the trocar therethrough, and said elastic layer 32 is continuous and overlies the region of said slit.

6. The medical catheter of any of the foregoing claims in combination with a trocar or the like (12), said combination being adapted for introduction into the body of a person, characterized in that, said trocar comprises an elongated, rigid shaft 18 sized to extend coaxially through said catheter, said trocar having a generally pointed head 20 at its distal end, and a reduced size portion 26 immediately proximal of said head, whereby, during insertion of said catheter by means of said trocar, there is no movement-impeding protrusion at the transition between said trocar head and the distal portion of the catheter.

7. The medical catheter and trocar combination of claim 6

characterized in that,

the distal surface of said trocar immediately proximal of said head 20 comprises a camming surface 28 disposed at an angle to the trocar axis to urge the end of the catheter to expand radially as said trocar is progressively withdrawn from the catheter.

8. The medical catheter and trocar combination of claim 6 or 7

characterized in that

the main body of the trocar shaft 18 is larger than said reduced size portion to define a neck 26 that provides a surface having a distally-directed component for applying insertion force to the end portion of said catheter.

9. The trocar of claim 7

characterized in that

wherein said camming surface is a surface 28 of revolution of distally increasing diameter to the head of said trocar.

10. The trocar of claim 6, 7 or 9

characterized in that

the largest diameter of said head and the diameter of the main body of the shaft are the same.

0150281

FIG 1

FIG 1a

FIG 2

FIG 3

FIG 4

FIG 5

## DOCUMENTS CONSIDERED TO BE RELEVANT

| Category | Citation of document with indication, where appropriate, of relevant passages | Relevant to claim | CLASSIFICATION OF THE APPLICATION (Int. Cl.4) |
|---|---|---|---|
| X | DE-A-1 809 364 (MEDIZINISCHE AKADEMIE CARL GUSTAV CARUS) <br><br> * Page 5, lines 16-22; figures 2,3 * <br><br> --- | 1,2, 6-10 | A 61 M 25/00 |
| X | DE-A-2 015 524 (D. SHERIDAN) <br><br> * Page 2, lines 21-26; figure 2 * <br><br> --- | 1,2, 6-10 | |
| A | US-A-3 030 953 (W. KOEHN) <br><br> * Figures 7,8 * <br><br> --- | 1,2 | |
| A | US-A-4 411 655 (D. SCHRECK) <br><br> * Abstract; figure 1 * <br><br> ----- | 6 | **TECHNICAL FIELDS SEARCHED (Int. Cl.4)** <br><br> A 61 M |

The present search report has been drawn up for all claims

| Place of search | Date of completion of the search | Examiner |
|---|---|---|
| THE HAGUE | 19-02-1985 | EHRSAM F. |